(19) Europäisches Patentamt
European Patent Office
Office européen des brevets

(11) **EP 4 760 611 A1**

(12) **DEMANDE DE BREVET EUROPEEN**

(43) Date de publication:
**17.06.2026   Bulletin 2026/25**

(21) Numéro de dépôt: **25222352.4**

(22) Date de dépôt: **10.12.2025**

(51) Classification Internationale des Brevets (IPC):
**G06Q 10/04** (2023.01)      **G06Q 50/40** (2024.01)
**G16H 50/20** (2018.01)      **G16H 50/30** (2018.01)
**G16H 40/63** (2018.01)

(52) Classification Coopérative des Brevets (CPC):
**G06Q 10/04; G06Q 50/40; G16H 40/63;
G16H 50/20; G16H 50/30**

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**
Etats d'extension désignés:
**BA**
Etats de validation désignés:
**GE KH LA MA MD TN**

(30) Priorité: **16.12.2024   FR 2414232**

(71) Demandeur: **AMPERE s.a.s.
92100 Boulougne-Billancourt (FR)**

(72) Inventeurs:
• **BASILIO, Numa
78084 Guyancourt (FR)**
• **LE-HIR, Nathalie
78084 Guyancourt (FR)**

(74) Mandataire: **Renault Group
1, avenue du Golf
FR TCR AVA 4 4C
78084 Guyancourt Cedex (FR)**

(54) **PROCÉDÉ DE GESTION DU MAL DES TRANSPORT ET SYSTÈME POUR LE METTRE EN OEUVRE**

(57)    Le procédé de comprend les étapes de : - fournir (E1) des algorithmes configurés pour déterminer une intensité de mal des transports théorique ; - traiter (E3) des données avec chaque algorithme pour en déduire une intensité de mal des transports théorique et une robustesse statistique associée ; - analyser (E5) lesdites intensités de mal des transports théoriques et lesdites robustesses statistiques pour en déduire une intensité de mal des transports la plus probable ainsi qu'une probabilité associée ; - déduire (E6) de ladite intensité la plus probable une mesure à prendre contre le mal des transports ; et - contrôler (E7, E8) l'exécution de ladite mesure en fonction de ladite probabilité associée.

[Fig. 2]

E1 → E2 → E3 → E4 → E5 → E6 → E7 → E8

**Description**

**Domaine technique**

La présente invention concerne, de manière générale, la gestion du mal des transports dans les véhicules.
Plus précisément, l'invention se rapporte à un procédé de gestion du mal des transport et à un système pour mettre en œuvre ce procédé de gestion dans un véhicule automobile.

**Techniques antérieures**

La recherche sur la détection du mal des transports en véhicule automobile ou simulateur de conduite est bien documentée et de nombreux algorithmes sont proposés, utilisant des données physiologiques des occupants du véhicule, combinées parfois à des données liées au mouvement du véhicule.
On connait par exemple de la demande internationale WO 2020/193127 un dispositif pour détecter le mal des transports d'une personne dans un véhicule configuré pour générer une valeur d'indice de mal des transports à partir de la réponse électrodermale et la température de la peau de la personne.
De manière générale, aucun système ne peut toutefois fournir une estimation de l'intensité du mal des transports suffisamment précise pour éviter les cas de faux positifs ou de faux négatifs.
Il subsiste donc un besoin d'améliorer la fiabilité de l'estimation de l'intensité de mal des transports.

**Exposé de l'invention**

L'invention vise en particulier à pallier cet inconvénient.
L'invention propose à cet effet un procédé de gestion du mal des transports au cours d'un trajet effectué avec un véhicule, comprenant les étapes de :

- fournir une pluralité d'algorithmes distincts chacun configuré pour déterminer une intensité de mal des transports théorique pour un occupant du véhicule en fonction d'au moins un paramètre prédéterminé ;
- collecter des données représentatives desdits paramètres prédéterminés ;
- traiter lesdites données collectées avec ladite pluralité d'algorithmes pour en déduire avec chaque algorithme une intensité de mal des transports théorique et une robustesse statistique associée à ladite intensité de mal des transports théorique ;
- analyser lesdites intensités de mal des transports théoriques et lesdites robustesses statistiques pour en déduire une intensité de mal des transports la plus probable pour l'occupant du véhicule ainsi que la probabilité associée à cette intensité la plus probable ;
- déduire de ladite intensité la plus probable au moins une mesure à prendre contre le mal des transports ; et
- contrôler l'exécution de ladite mesure à prendre en fonction de ladite probabilité associée.

Autrement dit, le procédé selon l'invention utilise une approche probabiliste pour évaluer l'intensité du mal des transports. En disposant de l'intensité de mal des transports ainsi que de la probabilité associée à cette intensité de mal des transports, on optimise non seulement la mesure à prendre contre le mal des transports mais aussi le contrôle du déclenchement de cette mesure.
Selon d'autres caractéristiques, l'étape de déduire une mesure à prendre contre le mal des transports comporte une étape d'identifier parmi un ensemble de mesures contre le mal des transports chacune associée à une plage d'intensités de mal des transports distincte celle dont la plage d'intensités de mal des transports associée inclut ladite intensité la plus probable, et dans lequel l'étape de contrôler l'exécution de ladite mesure à prendre comporte une étape de comparer ladite probabilité associée à un seuil de déclenchement.
Selon d'autres caractéristiques, l'étape d'analyser lesdites intensités de mal des transports théoriques et lesdites robustesses statistiques comprend l'utilisation d'un algorithme d'apprentissage configuré pour pondérer lesdites intensités de mal des transports théoriques, ledit algorithme d'apprentissage étant entraîné par des données d'apprentissage comprenant au moins une intensité de mal des transports réelle déclarée par un occupant dudit véhicule au cours d'un trajet préalable.
En outre, l'étape d'analyser lesdites intensités de mal des transports théoriques et lesdites robustesses statistiques comporte l'étape d'effectuer une moyenne pondérée desdites intensités de mal des transports théoriques et/ou desdites robustesses statistiques.
Avantageusement, l'algorithme d'apprentissage est un réseau de neurones.
Selon d'autres caractéristiques, au moins un dit algorithme est configuré pour déterminer une intensité de mal des transports théorique en fonction d'un paramètre lié au véhicule, et au moins un dit algorithme est configuré pour déterminer une intensité de mal des transports théorique en fonction d'un paramètre physiologique de l'occupant du véhicule.

Selon d'autres caractéristiques, au moins un dit algorithme est configuré pour déterminer une intensité de mal des transports théorique en fonction de la place de l'occupant dans le véhicule.

Selon d'autres caractéristiques, l'étape de traiter lesdites données collectées avec ladite pluralité d'algorithmes est répétée suivant un pas de temps variable déterminé en fonction de l'intensité de mal des transports la plus probable, de la probabilité associée et/ou de leur évolution dans le temps.

Selon d'autres caractéristiques, l'étape de traiter lesdites données collectées avec ladite pluralité d'algorithmes est répétée suivant un pas de temps variable déterminé en fonction du contexte de conduite et/ou de son évolution au cours du trajet effectué par le véhicule.

L'invention concerne en outre un système de gestion du mal des transports pour un véhicule, comportant des capteurs configurés pour fournir des données représentatives de paramètres prédéterminés, une interface homme-machine configurée pour fournir au moins une intensité de mal des transports réelle déclarée par un occupant dudit véhicule et un contrôleur configuré pour collecter auprès desdits capteurs et de ladite interface homme-machine lesdites données représentatives de paramètres prédéterminés et ladite intensité de mal des transports réelle pour mettre en œuvre les étapes du procédé tel que défini ci-dessus.

**Brève description des dessins**

D'autres buts, avantages et caractéristiques ressortiront de la description qui va suivre, donnée à titre purement illustratif et faite en référence aux dessins annexés sur lesquels :

La [Fig.1] illustre la structure d'un système configuré pour mettre en œuvre un procédé de gestion du mal des transports selon l'invention ; et
la [Fig.2] est un diagramme illustrant les étapes du procédé de gestion du mal des transports.

**DESCRIPTION DETAILLEE**

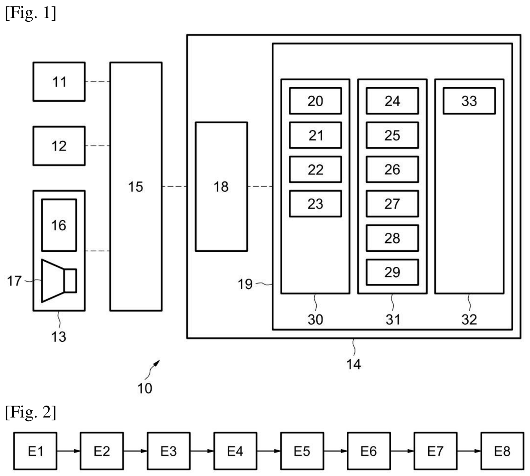

Le [Fig.1] illustre la structure d'un système 10 de gestion du mal des transports destiné à équiper un véhicule automobile (non illustré).

Le système 10 comporte un capteur 11 configuré pour fournir des données de mouvement du véhicule, un capteur 12 configuré pour fournir des données physiologiques d'un occupant du véhicule, une interface homme-machine 13, un contrôleur 14 et un bus de communication 15 auquel sont reliés les capteurs 11 et 12, l'interface 13 et le contrôleur 14.

Le capteur 11 est par exemple un capteur d'accélération mesurant l'accélération suivant les directions avant-arrière, gauche-droite et haut-bas conventionnellement définies par rapport au véhicule.

Le capteur 12 est par exemple un capteur de réponse électrodermale, mesurant la conductance électrique de la peau. La réponse électrodermale est un paramètre physiologique permettant d'évaluer l'intensité de la transpiration à la surface de la peau d'un individu, l'intensité de la transpiration étant, de manière connue, corrélée à celle d'un mal des transports que pourrait ressentir cet individu.

L'interface homme-machine 13 comporte ici un dispositif d'affichage interactif 16, par exemple un écran tactile, et un haut-parleur 17.

Le contrôleur 14, par exemple un calculateur du véhicule, comporte une unité de traitement de données 18 et une mémoire 19 connectée à l'unité de traitement 18.

Le bus de communication 15 est par exemple le bus CAN (pour *Controller Area Networken* anglais) du véhicule.

Le contrôleur 14 est configuré pour collecter les données fournies par les capteurs 11 et 12 ainsi que le dispositif d'affichage interactif 16, et pour enregistrer ces données dans la mémoire 19.

Le contrôleur 14 comporte en outre un ensemble de modules de gestion du mal des transports au cours d'un trajet effectué avec le véhicule, comprenant un premier module 30, un deuxième module 31 et un troisième module 32, ici chacun enregistrés dans la mémoire 19.

Le module 30 est configuré pour déterminer une intensité de mal des transports la plus probable $i_{prob}$ pour l'occupant du véhicule ainsi que la probabilité $P(i_{prob})$ associée à cette intensité $i_{prob}$.

Le module 30 comporte deux algorithmes distincts 20 et 21 pour la détermination d'intensités de mal des transports théoriques, un algorithme d'analyse 22 pour faire une analyse de probabilité des résultats fournis par les algorithmes 20 et 21, et un algorithme d'apprentissage 23 pour personnaliser l'algorithme d'analyse 22 à l'occupant du véhicule.

L'algorithme 20 est configuré pour traiter les données représentatives de l'accélération du véhicule collectées par le capteur 11 pour en déduire une première valeur d'intensité de mal des transports théorique $i_{th1}$ ainsi qu'une première

robustesse statistique $r_1$ associée à cette première valeur d'intensité de mal des transports théorique $i_{th1}$.

L'algorithme 20 est ici basé sur les relations :

$$i_{th1} = \alpha \cdot a_{rms} + \beta$$

$$r_1 = \frac{\sigma_{a_{rms}}}{a_{rms}}$$

où $\alpha$ et $\beta$ sont des coefficients obtenus par apprentissage, $a_{rms}$ est la moyenne quadratique des composantes $a_x(t)$, $a_y(t)$, et $a_z(t)$ de l'accélération mesurée par le capteur 11 suivant les directions avant-arrière, gauche-droite et haut-bas respectivement, pendant une durée $T$, à savoir :

$$a_{rms} = \sqrt{\frac{1}{T}\int_0^T \left( a_{\tilde{x}}^2(t) + a_{\tilde{y}}^2(t) + a_{\tilde{z}}^2(t) \right) dt}$$

et $\sigma a_{rms}$ est l'écart-type des valeurs d'accélération.

Par exemple, si $a_{rms} = 2.5\ m/s^2$, $a = 0.8$, $\beta = 1$ et $\sigma a_{rms} = 0.5$, alors :

$$i_{th1} = 0.8 \cdot 2.5 + 1 = 3.0$$

$$r_1 = \frac{0.5}{2.5} = 0.2$$

L'algorithme 21 est configuré pour traiter les données représentatives de la réponse électrodermale collectées par le capteur 12 pour en déduire une deuxième valeur d'intensité de mal des transports théorique $i_{th2}$ ainsi qu'une deuxième robustesse statistique $r_2$ associée à cette deuxième valeur d'intensité de mal des transports théorique $i_{th2}$.

L'algorithme 21 est ici basé sur les relations :

$$i_{th2} = \gamma \cdot max\left(G_{fast}\right) + \delta \cdot G_{slow}$$

$$r_2 = \frac{std(G_{fast})}{max(G_{fast})}$$

où $\gamma$ et $\delta$ sont des coefficients obtenus par apprentissage, $G_{fast}(t)$ et $G_{slow}(t)$ sont respectivement la variation rapide et la variation lente extraites d'un même signal brut $GSR\ (t)$ fourni par le capteur 12 (GSR étant l'acronyme de l'expression en anglais *Galvanic Skin Response),* $std$ une fonction renvoyant l'écart-type et $max$ une fonction renvoyant un maximum.

Par exemple, si $max(G_{fast}) = 3.5\ \mu S$, $G_{slow} = 1.2\ \mu S$, $\gamma = 0.7$, $\delta = 0.3$ et $std\ (G_{fast}) = 0.4$, alors :

$$i_{th2} = 0.7 \cdot 3.5 + 0.3 \cdot 1.2 = 2.45 + 0.36 = 2.81$$

$$r_2 = \frac{0.4}{3.5} = 0.114$$

On notera que les algorithmes 20 et 21 se distinguent ici par le paramètre mesuré à partir duquel ils déterminent chacun l'intensité de mal des transports théorique, à savoir ici l'accélération du véhicule et la réponse électrodermale de l'occupant du véhicule.

On notera que les algorithmes 20 et 21 se distinguent ici en outre par la relation, ici empirique, qu'ils établissent chacun entre le paramètre mesuré et l'intensité de mal des transports théorique $i_{th1}$ ou $i_{th2}$ correspondante.

En variante, les algorithmes 20 et 21 pourraient ne se distinguer que par la relation qu'ils établissent chacun entre le paramètre mesuré et l'intensité de mal des transports théorique correspondante, le paramètre mesuré étant le même pour chaque algorithme.

L'algorithme d'analyse 22 est configuré pour effectuer une étape d'analyse de probabilité des valeurs d'intensités de mal des transports théoriques $i_{th1}$, $i_{th2}$ ainsi que des robustesses statistiques $r_1$, $r_2$ déterminées par les algorithmes 20 et 21, pour en déduire l'intensité de mal des transports la plus probable $i_{prob}$ ainsi que la probabilité associée $P(i_{prob})$.

Au cours de l'étape d'analyse de probabilité, des coefficients de pondération $k_1$ et $k_2$ sont affectés à une intensité de mal des transports théorique $i_{th1}$, $i_{th2}$ respectivement.

L'algorithme 22 est ici basé sur les relations :

$$i_{prob} = \frac{k_1 i_{th1} + k_2 i_{th2}}{k_1 + k_2}$$

où

$$k_1 = \frac{1}{r_1} \text{ et } k_2 = \frac{1}{r_2},$$

et

$$P(i_{prob}) = 1 - \frac{|i_{th1} - i_{th2}|}{max(i_{th1}, i_{th2})}$$

Autrement dit, l'étape d'analyse de probabilité inclut la détermination de l'intensité de mal des transports la plus probable $i_{prob}$ comme étant égale à la moyenne pondérée des valeurs d'intensité théoriques $i_{th1}$, $i_{th2}$ en utilisant les coefficients de pondération $k_1$, $k_2$.

La probabilité associée $P(i_{prob})$ est quant à elle, ici, une probabilité de similarité des valeurs d'intensité théoriques $i_{th1}$, $i_{th2}$.

Par exemple, avec les valeurs calculées ci-dessus pour $i_{th1}$, $r_1$, $i_{th2}$ et $r_2$, on obtient :

$$k_1 = \frac{1}{0.2} = 5$$

$$k_2 = \frac{1}{0.114} \approx 8.77$$

$$i_{prob} = \frac{53.0 + 8.77 \cdot 2.81}{5 + 8.77} = \frac{15 + 24.63}{13.77} \approx 2.89$$

$$P(i_{prob}) = 1 - \frac{|3.0 - 2.81|}{3.0} = 1 - \frac{0.19}{3.0} \approx 0.937$$

L'algorithme d'apprentissage 23, ici un réseau de neurones, est configuré pour ajuster les coefficients de pondération $k_1$, $k_2$ en fonction de données d'apprentissage collectées au cours d'au moins un trajet préalable effectué par l'occupant avec le véhicule.

Les données d'apprentissage comportent, pour chaque trajet préalable, au moins une intensité de mal des transports la plus probable $i_{prob}$, les données des capteurs 11 et 12 à partir desquelles a été déterminée cette intensité $i_{prob}$ et au moins une intensité de mal des transports réelle $i_{dec}$ déclarée par l'occupant du véhicule. L'intensité de mal des transports réelle $i_{dec}$ est par exemple déclarée en utilisant le dispositif d'affichage interactif 16, ou un dispositif à commande vocale.

L'algorithme d'apprentissage 23 est ici configuré pour mettre en œuvre un ajustement par rétropropagation pour minimiser la valeur $|i_{prob} - i_{dec}|$ en ajustant les coefficients de pondération $k_1$ et $k_2$. L'algorithme d'apprentissage 23 est basé sur la fonction de perte :

$$L = \left( i_{prob} - i_{dec} \right)^2$$

L'algorithme d'apprentissage 23 ajuste les coefficients $k_1$ et $k_2$ pour minimiser cette perte, affinant la correspondance entre les intensités de mal des transports calculées et les intensités de mal des transports déclarées.

Le module 31 est configuré pour déduire de l'intensité la plus probable $i_{prob}$ une mesure adaptée contre le mal des transports et pour fournir des instructions de mise en œuvre de cette mesure au contrôleur 14.

Le module 31 comporte à cet effet une pluralité de blocs d'instructions, ici cinq blocs 24, 25, 26, 27 et 28, ainsi qu'un algorithme de sélection 29 configuré pour identifier un bloc d'instructions 24 à 28 à exécuter en fonction de l'intensité la plus probable $i_{prob}$.

Chaque bloc d'instructions 24 à 28 est associé à une plage de valeurs $pvi_{b24}$, $pvi_{b25}$, $pvi_{b26}$, $pvi_{b27}$ et $pvi_{b28}$ de l'intensité du mal des transports et permet au contrôleur 14 de mettre en œuvre une mesure contre le mal des transports adaptée à la plage de valeurs d'intensités correspondante.

Les plages de valeurs $pvi_{b24}$ à $pvi_{b28}$ sont par exemple respectivement [0 ; 2[, [2 ; 4[, [4 ; 6[, [6 ; 8[ et [8 ; 10], les valeurs 0, 2, 4, 6, 8 et 10 correspondant respectivement à une intensité de mal des transports nulle, faible, moyenne, élevée, très élevée et maximale sur l'échelle d'inconfort allant de 0 à 10.

L'algorithme de sélection 29 est configuré pour trouver parmi les blocs d'instructions 24 à 28 celui dont la plage de valeurs $pvi_{b24}$ à $pvi_{b28}$ associée inclut la valeur d'intensité de mal des transports $i_{prob}$, et pour générer un identifiant pour ce bloc d'instruction.

Par exemple, avec la valeur $i_{prob}$ = 2.89 calculée plus haut, l'algorithme 29 sélectionnera le bloc d'instructions 25.

Le module 32 est configuré pour contrôler l'exécution du bloc d'instructions à exécuter en fonction de la probabilité associée $P(i_{prob})$.

Le module 32 comporte à cet effet un algorithme de déclenchement 33 configuré pour comparer la probabilité $P(i_{prob})$ avec un seuil de déclenchement $s_{decl}$ et générer une valeur binaire de contrôle du déclenchement, par exemple égale 1 si la probabilité $P(i_{prob})$ est supérieure ou égale au seuil de déclenchement $s_{dec1}$, et égale à 0 sinon. Le seuil de déclenchement $s_{decl}$ est par exemple égal à 0,8. Compte-tenu de la valeur de $P(i_{prob})$ = 0.937 calculée ci-dessus, l'algorithme de déclenchement 33 fixera la valeur binaire de contrôle du déclenchement à 1.

Le contrôleur 14 est configuré pour exécuter le bloc d'instructions identifiée par le module 31 si la valeur de contrôle générée par le module 32 est égale à 1.

La [Fig.2] est un diagramme illustrant des étapes d'un procédé de gestion du mal des transports mises en œuvre par le système 10 au cours d'un trajet effectué avec le véhicule.

Au cours d'une étape E1, les algorithmes 20 et 21 sont fournis en étant enregistrés dans la mémoire 19 du contrôleur 14. Les algorithmes 20 et 21 sont par exemple retrouvés dans de la littérature technique disponible sur une base de données en ligne, telle qu'une base brevet.

Au cours d'une étape E2, le contrôleur 14 collecte les données représentatives de l'accélération du véhicule et de la réponse électrodermale de l'occupant du véhicule fournies par les capteurs 11 et 12 respectivement, et les enregistrent dans la mémoire 19.

Au cours d'une étape E3, l'unité de traitement de données 18 traite les données obtenues à l'étape E2 avec chacun des algorithmes 20 et 21 pour en déduire la première valeur d'intensité de mal des transports théorique $i_{th1}$, la première robustesse statistique $r_1$, la deuxième valeur d'intensité de mal des transports théorique $i_{th2}$ et la deuxième robustesse statistique $r_2$, les valeurs $i_{th1}$, $r_1$, $i_{th2}$ et $r_2$ étant enregistrées dans la mémoire 19.

L'étape E3 est ici répétée suivant un pas de temps fixe, de l'ordre de 3 minutes, les valeurs $i_{th1}$, $r_1$, $i_{th2}$ et $r_2$ enregistrées dans la mémoire 19 étant mises à jour à chaque itération.

Au cours d'une étape E4, l'unité de traitement de données 18 utilise l'algorithme d'apprentissage 23 pour affecter les coefficients $k_1$ et $k_2$ aux valeurs $i_{th1}$, $i_{th2}$ respectivement et/ou aux valeurs $r_1$, $r_2$ respectivement et met en conséquence à jour les valeurs $i_{th1}$, $r_1$, $i_{th2}$ et $r_2$ enregistrées dans la mémoire 19.

Au cours d'une étape E5, l'unité de traitement de données 18 utilise l'algorithme d'analyse 22 pour effectuer une analyse de probabilité avec les valeurs $i_{th1}$, $r_1$, $i_{th2}$ et $r_2$ retrouvées dans la mémoire 19 et en déduit la valeur d'intensité de mal des transports la plus probable $i_{prob}$ pour l'occupant du véhicule ainsi que la probabilité associée $P(i_{prob})$, puis enregistre les valeurs $i_{prob}$ et $P(i_{prob})$ dans la mémoire 19.

Au cours d'une étape E6, l'unité de traitement de données 18 utilise l'algorithme de sélection 29 pour trouver parmi les blocs d'instructions 24 à 28 celui dont la plage de valeurs $pvi_{b24}$ à $pvi_{b28}$ associée inclut la valeur d'intensité de mal des transports $i_{prob}$, et génère un identifiant pour ce bloc d'instruction. L'unité de traitement de données 18 enregistre ensuite l'identifiant dans la mémoire 19.

On notera que cette étape E6 revient en pratique à déduire de l'intensité de mal des transports la plus probable, définie par sa valeur $i_{prob}$, une mesure à prendre contre le mal des transports, définie par son bloc d'instructions correspondant.

Au cours d'une étape E7, l'unité de traitement de données 18 utilise l'algorithme de déclenchement 33 pour comparer la probabilité $P(i_{prob})$ avec le seuil de déclenchement $s_{decl}$ et génère une valeur binaire de contrôle du déclenchement, par exemple égale 1 si la probabilité $P(i_{prob})$ est supérieure ou égale au seuil de déclenchement $s_{decl}$, et égale à 0 sinon. L'unité de traitement de données 18 enregistre la valeur de contrôle du déclenchement dans la mémoire 19.

Au cours d'un étape E8, le contrôleur 14 consulte la mémoire 19 pour y retrouver l'identifiant du bloc d'instruction ainsi que la valeur binaire de contrôle et exécute les instructions contenues dans le bloc d'instructions si la valeur binaire de contrôle est égale à 1.

On notera que les étapes E7 et E8 reviennent en pratique à contrôler l'exécution de la mesure à prendre contre le mal des transports identifiée à l'étape E6 en fonction de la probabilité associée $P(i_{prob})$.

La mesure contre le mal des transports associée au bloc d'instructions 24 (intensité de mal des transports de 0 à 2) comporte ici l'action d'arrêter une autre mesure contre le mal des transports éventuellement en cours d'exécution.

La mesure contre le mal des transports associée au bloc d'instructions 25 (intensité de mal des transports de 3 ou 4) est une mesure contre le mal des transports préventive, comportant par exemple l'action d'émettre des consignes sonores avec le haut-parleur 17 en vue d'anticiper des contextes de conduite risquant d'aggraver le mal des transports, tel qu'une route sinueuse.

La mesure contre le mal des transports associée au bloc d'instructions 26 (intensité de mal des transports de 5 ou 6) est ici une mesure contre le mal des transports corrective, comportant par exemple l'émission d'un son binaural avec le haut-parleur 17, l'abaissement de la température dans l'habitacle du véhicule et/ou l'activation d'une ventilation.

Dans le mode de réalisation illustré, l'étape E3 est répétée suivant un pas de temps fixe. En variante, le pas de temps est variable afin d'optimiser les sollicitations de la puissance de calcul du contrôleur 14. Le pas de temps variable peut être déterminé en fonction de l'intensité de mal des transports la plus probable $i_{prob}$, de la probabilité associée $P(i_{prob})$ et/ou de l'évolution dans le temps de ces valeurs $i_{prob}$ et $P(i_{prob})$. Par exemple, si l'intensité $i_{prob}$ augmente à chaque itération alors que la probabilité $P(i_{prob})$ reste élevée, alors le pas de temps sera diminué, par exemple à 1 minute. Au contraire, le pas de temps pourra être augmenté, par exemple à 10 minutes, si la probabilité $P(i_{prob})$ reste faible pendant plusieurs itérations. En variante encore, le pas de temps variable est déterminé en fonction de données représentatives du contexte de conduite et/ou de son évolution au cours du trajet effectué par le véhicule. De telles données représentatives du contexte de conduite comprennent par exemple le type de route (route de ville, route de campagne, autoroute etc.), la température extérieure, la température à l'intérieur de l'habitacle, l'altitude etc. En variante encore, le pas de temps variable est déterminé en fonction du temps de trajet écoulé, par exemple est diminué au fur et à mesure que le temps de trajet écoulé augmente.

Dans un mode de réalisation, les données d'apprentissage peuvent en outre comporter un identifiant propre à l'occupant du véhicule ayant déclarée l'intensité $i_{dec}$. Ainsi, l'algorithme d'apprentissage 23 peut être configuré pour distinguer les données d'apprentissage propres à un occupant du véhicule de celles propres à un autre occupant du véhicule et générer des coefficients de pondération $k_1$, $k_2$ propres à chacun des occupants.

Dans un mode de réalisation, le procédé de gestion ne comporte pas l'étape d'utiliser un algorithme d'apprentissage.

Dans un mode de réalisation, l'étape E6 de déduire une mesure à prendre contre le mal des transports de l'intensité de mal des transports la plus probable n'est effectuée que si la probabilité $P(i_{prob})$ est supérieure au seuil de déclenchement $s_{dec1}$.

Dans un mode de réalisation, le module 31 comporte plus ou moins que cinq blocs d'instructions, par exemple trois blocs, ou sept blocs.

Dans un mode de réalisation, l'algorithme 20 et/ou l'algorithme 21 est en outre configuré pour déterminer l'intensité de mal des transports théorique en fonction de la place de l'occupant dans le véhicule. Par exemple, l'algorithme 20 et/ou l'algorithme 21 applique un coefficient de majoration si la place de l'occupant est une place de passager.

Dans un mode de réalisation, le premier module 30 comporte plus que deux algorithmes distincts, tels que 20 et 21, pour la détermination d'une intensité de mal des transports théorique, par exemple 3, 10 ou 100 algorithmes.

Dans un mode de réalisation, au moins l'un des algorithmes tels que 20 ou 21 est configuré pour déterminer une intensité de mal des transports théorique pour un occupant du véhicule en fonction d'une pluralité de paramètres prédéterminés, les paramètres prédéterminés étant sélectionnés parmi :

- des paramètres physiologiques de l'occupant du véhicule, tels que la température corporelle, la température cutanée, la réponse électrodermale, la fréquence respiratoire, le taux d'oxygène dans l'air expiré, le taux de dioxygène dans l'air expiré, la fréquence cardiaque et/ou le rythme cardiaque;
- un paramètre identifiant la place de l'occupant dans le véhicule, telle que la place conducteur ou une place passager située à l'avant, à l'arrière, à gauche, à droite ou au milieu ;
- des paramètres de mouvement du véhicule, tels que la vitesse, l'accélération et/ou la vibration ;
- des paramètres atmosphériques, tels que la température de l'air, le taux d'oxygène, le taux d'anions d'oxygène, le taux de dioxygène et/ou le niveau sonore ;

- des paramètres géographiques, tels que l'altitude et/ou des coordonnées GPS ;
- d'autres paramètres tels que le type de route empruntée par le véhicule (route de ville, route de campagne, autoroute etc.), la température extérieure et/ou la température à l'intérieur de l'habitacle ; le système comportant alors, à la place des capteurs 11 et 12 ou en complément des capteurs 11 et 12, d'autres capteurs configurés pour collecter des données représentatives de ces paramètres prédéterminés, par exemple le capteur 11 est remplacé ou complété par des capteurs de vitesse, de vibration ou de l'angle du volant, tandis que le capteur 12 est remplacé ou complété par des capteurs de température du corps, de la fréquence cardiaque, de la concentration en oxygène et/ou en CO2 dans l'air expiré.

On notera que les paramètres de mouvement du véhicule font plus généralement partie des paramètres liés au véhicule, ces derniers pouvant comporter en outre, par exemple, le régime du moteur, l'angle du volant, l'angle de dossier des sièges, l'état activé ou désactivé du système ABS, la température de consigne de la climatisation, la température dans l'habitacle, l'état ouvert ou fermé des fenêtres etc.

Dans un mode de réalisation, le véhicule équipé avec le système de gestion du mal des transports est sélectionné parmi un véhicule terrestre, un aéronef et un navire, le véhicule étant réel ou virtuel.


**Revendications**

1.  Procédé de gestion du mal des transports au cours d'un trajet effectué avec un véhicule, **caractérisé en ce qu'**il comprend les étapes de :

    - fournir (E1) une pluralité d'algorithmes distincts (20, 21) chacun configuré pour déterminer une intensité de mal des transports théorique pour un occupant du véhicule en fonction d'au moins un paramètre prédéterminé ;
    - collecter (E2) des données représentatives desdits paramètres prédéterminés ;
    - traiter (E3) lesdites données collectées avec ladite pluralité d'algorithmes pour en déduire avec chaque algorithme (20, 21) une intensité de mal des transports théorique et une robustesse statistique associée à ladite intensité de mal des transports théorique ;
    - analyser (E5) lesdites intensités de mal des transports théoriques et lesdites robustesses statistiques pour en déduire une intensité de mal des transports la plus probable pour l'occupant du véhicule ainsi que la probabilité associée à cette intensité la plus probable ;
    - déduire (E6) de ladite intensité la plus probable au moins une mesure à prendre contre le mal des transports ; et
    - contrôler (E7, E8) l'exécution de ladite mesure à prendre en fonction de ladite probabilité associée.

2.  Procédé selon la revendication 1, dans lequel l'étape de déduire (E6) une mesure à prendre contre le mal des transports comporte une étape d'identifier parmi un ensemble de mesures contre le mal des transports chacune associée à une plage d'intensités de mal des transports distincte celle dont la plage d'intensités de mal des transports associée inclut ladite intensité la plus probable, et dans lequel l'étape de contrôler l'exécution de ladite mesure à prendre comporte une étape de comparer ladite probabilité associée à un seuil de déclenchement.

3.  Procédé selon la revendication 1 ou 2, dans lequel l'étape d'analyser (E5) lesdites intensités de mal des transports théoriques et lesdites robustesses statistiques comprend l'utilisation (E4) d'un algorithme d'apprentissage (23) configuré pour pondérer lesdites intensités de mal des transports théoriques, ledit algorithme d'apprentissage (23) étant entraîné par des données d'apprentissage comprenant au moins une intensité de mal des transports réelle déclarée par un occupant dudit véhicule au cours d'un trajet préalable.

4.  Procédé selon la revendication 3, dans lequel l'étape d'analyser (E5) lesdites intensités de mal des transports théoriques et lesdites robustesses statistiques comporte l'étape d'effectuer une moyenne pondérée desdites intensités de mal des transports théoriques.

5.  Procédé selon la revendication 3 ou 4, dans lequel l'algorithme d'apprentissage (23) est un réseau de neurones.

6.  Procédé selon l'une quelconque des revendications 1 à 5, dans lequel au moins un dit algorithme (20) est configuré pour déterminer une intensité de mal des transports théorique en fonction d'un paramètre lié au véhicule, et au moins un dit algorithme (21) est configuré pour déterminer une intensité de mal des transports théorique en fonction d'un paramètre physiologique de l'occupant du véhicule.

7.  Procédé selon l'une quelconque des revendications 1 à 6, dans lequel au moins un dit algorithme est configuré pour déterminer une intensité de mal des transports théorique en fonction de la place de l'occupant dans le véhicule.

8. Procédé selon l'une quelconque des revendications 1 à 7, dans lequel l'étape de traiter (E3) lesdites données collectées avec ladite pluralité d'algorithmes (20, 21) est répétée suivant un pas de temps variable déterminé en fonction de l'intensité de mal des transports la plus probable, de la probabilité associée et/ou de leur évolution dans le temps.

9. Procédé selon l'une quelconque des revendications 1 à 8, dans lequel l'étape (E3) de traiter lesdites données collectées avec ladite pluralité d'algorithmes (20, 21) est répétée suivant un pas de temps variable déterminé en fonction du contexte de conduite et/ou de son évolution au cours du trajet effectué par le véhicule.

10. Système (10) de gestion du mal des transports pour un véhicule, comportant des capteurs (11, 12) configurés pour fournir des données représentatives de paramètres prédéterminés, une interface homme-machine (13) configurée pour fournir au moins une intensité de mal des transports réelle déclarée par un occupant dudit véhicule, et un contrôleur (14) configuré pour collecter auprès desdits capteurs (11, 12) et de ladite interface homme-machine (13) lesdites données représentatives de paramètres prédéterminés et ladite intensité de mal des transports réelle pour mettre en œuvre les étapes du procédé selon l'une quelconque des revendications 4 à 9 chacune selon la revendication 3.

[Fig. 1]

[Fig. 2]

Europäisches
Patentamt

European
Patent Office

Office européen
des brevets

# RAPPORT DE RECHERCHE EUROPEENNE

**Numéro de la demande**

EP 25 22 2352

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2022/001893 A1 (TARTZ ROBERT [US]) 6 janvier 2022 (2022-01-06) * abrégé * * alinéas [0002] - [0005] * * alinéas [0030] - [0057]; figures 1-3 * * alinéas [0101] - [0108]; figure 9 * * alinéa [0077] * * alinéas [0114], [0125] * ----- | 1-10 | INV. G06Q10/04 G06Q50/40 G16H50/20 G16H50/30 G16H40/63 |
| A | US 2019/022347 A1 (WAN JINGYAN [US] ET AL) 24 janvier 2019 (2019-01-24) * abrégé * * alinéas [0004] - [0023] * ----- | 1-10 | |
| A | US 2024/181827 A1 (ANDERSON ZACKARY MARTIN [US] ET AL) 6 juin 2024 (2024-06-06) * abrégé * * alinéas [0004] - [0023] * * alinéa [0082] * ----- | 1-10 | |
| A | US 2021/031789 A1 (MORIURA YUTA [JP] ET AL) 4 février 2021 (2021-02-04) * alinéas [0045] - [0066] * * alinéas [0002] - [0009] * ----- | 1-10 | **DOMAINES TECHNIQUES RECHERCHES (IPC)** G06Q G16H |
| A | US 2020/324073 A1 (RAJAN KESAVELU SHEKAR PRAMOD [IN] ET AL) 15 octobre 2020 (2020-10-15) * abrégé * * alinéas [0004] - [0024] * * alinéa [0095] * ----- | 3-5,8,9 | |

Le présent rapport a été établi pour toutes les revendications

| Lieu de la recherche | Date d'achèvement de la recherche | Examinateur |
|---|---|---|
| Munich | 13 avril 2026 | Dedek, Frédéric |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

...........................................................................................................

& : membre de la même famille, document correspondant

EPO FORM 1503 03.82 (P04C02)

**EP 4 760 611 A1**

## ANNEXE AU RAPPORT DE RECHERCHE EUROPEENNE
## RELATIF A LA DEMANDE DE BREVET EUROPEEN NO.

EP 25 22 2352

13-04-2026

| Document brevet cité au rapport de recherche | Date de publication | Membre(s) de la famille de brevet(s) | Date de publication |
|---|---|---|---|
| US 2022001893 A1 | 06-01-2022 | BR 112022026184 A2 | 17-01-2023 |
| | | CN 115768671 A | 07-03-2023 |
| | | EP 4175860 A1 | 10-05-2023 |
| | | KR 20230034973 A | 10-03-2023 |
| | | PH 12022553495 A1 | 22-04-2024 |
| | | TW 202218623 A | 16-05-2022 |
| | | US 2022001893 A1 | 06-01-2022 |
| | | WO 2022005587 A1 | 06-01-2022 |
| US 2019022347 A1 | 24-01-2019 | AUCUN | |
| US 2024181827 A1 | 06-06-2024 | CN 107848360 A | 27-03-2018 |
| | | EP 3303025 A1 | 11-04-2018 |
| | | EP 3888958 A1 | 06-10-2021 |
| | | EP 4242054 A2 | 13-09-2023 |
| | | JP 6578024 B2 | 18-09-2019 |
| | | JP 7197621 B2 | 27-12-2022 |
| | | JP 2018524223 A | 30-08-2018 |
| | | JP 2019218055 A | 26-12-2019 |
| | | JP 2021113046 A | 05-08-2021 |
| | | JP 2023027286 A | 01-03-2023 |
| | | KR 20180033139 A | 02-04-2018 |
| | | KR 20200035498 A | 03-04-2020 |
| | | KR 20220042253 A | 04-04-2022 |
| | | KR 20230010777 A | 19-01-2023 |
| | | KR 20240172771 A | 10-12-2024 |
| | | US 2017136842 A1 | 18-05-2017 |
| | | US 2018162186 A1 | 14-06-2018 |
| | | US 2020055362 A1 | 20-02-2020 |
| | | US 2022126640 A1 | 28-04-2022 |
| | | US 2024181827 A1 | 06-06-2024 |
| | | WO 2016197068 A1 | 08-12-2016 |
| US 2021031789 A1 | 04-02-2021 | CN 111867459 A | 30-10-2020 |
| | | EP 3767606 A1 | 20-01-2021 |
| | | JP 7228765 B2 | 27-02-2023 |
| | | JP WO2019177002 A1 | 08-04-2021 |
| | | US 2021031789 A1 | 04-02-2021 |
| | | WO 2019177002 A1 | 19-09-2019 |
| US 2020324073 A1 | 15-10-2020 | EP 3725354 A1 | 21-10-2020 |
| | | US 2020324073 A1 | 15-10-2020 |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2020193127 A